# EUROPEAN PATENT SPECIFICATION

(11) **EP 3 744 264 B1**
(45) Date of publication and mention of the grant of the patent: **29.05.2024**
(21) Application number: 19761116.3
(22) Date of filing: 27.02.2019
(51) Int. Cl.: A61B 17/00, A61B 17/12

(54) **OCCLUDER, AND MEDICAL DEVICE**
OKKLUDER UND MEDIZINISCHE VORRICHTUNG
DISPOSITIF D'OCCLUSION ET DISPOSITIF MÉDICAL

(30) Priority: 02.03.2018 CN 201810176022
(43) Date of publication of application: 02.12.2020
(73) Proprietor: Shanghai Microport Cardioadvent Co., Ltd., Shanghai 201203 (CN)
(72) Inventor: ZHOU, Yi, Shanghai 201203 (CN); ZHOU, Ting, Shanghai 201203 (CN); YAO, Yao, Shanghai 201203 (CN); LI, Junfei, Shanghai 201203 (CN)
(74) Representative: Marks & Clerk LLP
(86) International application number: PCT/CN2019/076244
(87) International publication number: WO 2019/165966

(56) References cited:
- WO-A1-2012/003317
- WO-A1-2015/189307
- WO-A1-2017/214577
- CN-A- 106 344 100
- CN-A- 107 126 240
- CN-A- 107 233 114
- CN-A- 107 233 116
- CN-B- 104 688 292
- CN-U- 204 839 624
- US-A1- 2006 135 947
- US-A1- 2014 257 373
- US-A1- 2014 330 299

## Description

### TECHNICAL FIELD

The present application relates to the field of medical instruments and, in particular, to an occluder and a medical device.

### BACKGROUND

At present, an occluder product available on the market is loaded into a sheath by elongating and flattening the occluder to a suitable size taking advantage of the product's material and structural elasticity. This approach suffers from two inherent shortcomings: (i) the occluder can barely restore the exact original dimension after it is released; and (ii) the elongated occluder requires a greater sheath length when released, which is unfavorable to surgical operations. For example, during the implantation of a left atrial appendage (LAA) occluder, an occluder-loading sheath section must be inserted into the LAA. However, since LAAs vary in shape and depth, when the inserted sheath section is excessively long, it may damage or even pierce tissues. Therefore, the scope of application of a device is limited by the length of the LAA in the sheath-the shorter the length of the sheath occupied by LAA, the more shapes of LAA the occluder is applicable to.

US2014/257373A1 discloses a medical device including a tubular member having a proximal end and a distal end, a tether comprising a first end and a second end, wherein the first end of the tether is coupled to the proximal end or the distal end of the tubular member. An engagement member is coupled to the second end of the tether and disposed at least partially within the tubular member and between the proximal and distal ends of the tubular member, when the tubular member is in a relaxed configuration.

WO 2012/003317A1 discloses a left atrial appendage occlusion device including an occluder disk, a middle portion including a coiled element, a first anchoring element and a self-centering element that connects to the occluder disk and middle portion.

US2006/135947A1 discloses a means for occluding body passageways, particularly lung passageways. This is achieved using occlusal stents delivered with the use of any suitable delivery system, particularly a minimally invasive approach with instruments introduced through the mouth. A target lung tissue segment is isolated from other regions of the lung by deploying an occlusal stent into a target area of a lung passageway. A variety of different occlusal stent designs are provided.

Conventional methods for improving dimension retention include structural design improvements and material heat treatment optimization. The problem of excessive in-sheath occluder length has generally to be solved through structural design modifications. Such approaches are all at the price of other aspects of performance, which places great limitations on the structural design of occluders. Therefore, those skilled in the art are always troubled by the problem of excessive in-sheath occluder lengths.

### SUMMARY

The invention is defined in independent claim 1. Certain optional features of the invention are defined in the dependent claims.

The aim of the present application to provide an occluder and a medical
device to solve the problem that excessive in-sheath length of the existing occluder brings about various inconveniences to its use.

To solve the above problem, the present application provides an occluder, which comprises:
an occlusion member having a distal end and a proximal end; and
a limiting member connected to each of the distal and proximal ends and configured to limit an elongating distance between the distal and proximal ends, characterized in that, the limiting member is a telescopic structure and has its two ends connected to the distal and proximal ends respectively, and wherein the telescopic structure is a nested tube formed by multiple metal tube sections that are nested one into the other.

Optionally, the occluder further comprises a hollow structure, the limiting member being located in a chamber of the hollow structure, the hollow structure being located in the occlusion member.

Optionally, in the occluder, the hollow structure is a spring or a telescopic tube.

Optionally, the occluder further comprises at least one deflecting member, through which at least one end of the limiting member is connected to the distal or proximal end.

Optionally, in the occluder, the limiting member is connected to the distal and proximal ends by means of suturing, bonding, hot-melting, welding, threading or snapping.

Optionally, in the occluder, the occlusion member comprises a frame covered with a membrane, wherein the distal and proximal ends are on the frame.

The present application also provides a medical device, which comprises:
a sheath defining a delivery channel; and
the occluder as defined above. The occluder is able to reach a target site through the delivery channel. When the occluder is loaded in the sheath, the occlusion member in the occluder is in a partially or fully folded configuration, and the limiting member in the occluder is tightened to limit the elongating distance between the distal and proximal ends of the occlusion member. When the occluder is released from the sheath, the occlusion member is in a deployed configuration, and the limiting member is in an unconstrained state.

In the occluder and medical device provided in the present application, the occluder comprises the limiting member connected to each of the distal and proximal ends of the occlusion member and configured to limit the elongating distance between the distal and proximal ends. As a result, the occluder is in a partially- or fully-folded configuration when loaded in the sheath, thereby avoiding the excessive in-sheath length of the occluder. Further, since the occluder in the sheath is not fully elongated and is in a partially or fully folded configuration, the dimension of the occluder after it is released from the sheath is better maintained.

### BRIEF DESCRIPTION OF THE DRAWINGS

Fig. 1 is a schematic illustration of an occluder in a deployed configuration according to embodiment 1 which shares many features with the present invention but which does not fall within the scope of the claimed invention.
Fig. 2 is a schematic illustration of the occluder in a folded configuration according to embodiment 1 which does not fall within the scope of the present invention.
Fig. 3 is a schematic illustration of the occluder in an elongated configuration according to embodiment 1 which does not fall within the scope of the present invention.
Fig. 4 is a schematic illustration of an occluder in a deployed configuration according to embodiment 3 which shares many features with the present invention but which does not fall within the scope of the claimed invention.
Fig. 5 is a schematic illustration of the occluder in a folded configuration according to embodiment 3 which does not fall within the scope of the present invention.
Fig. 6 is a schematic illustration of an occluder in a deployed configuration according to embodiment 5 which shares many features with the present invention but which does not fall within the scope of the claimed invention.
Fig. 7 is a schematic illustration of the occluder in a folded configuration according to embodiment 5 which does not fall within the scope of the present invention.
Fig. 8 is a schematic illustration of an occluder in a deployed configuration according to embodiment 6 of the present application.
Fig. 9 is a schematic illustration of the occluder in a folded configuration according to embodiment 6 of the present application.

In these figures,
100, occluder; 110, occlusion member; 111, distal end; 112, proximal end; 120, limiting member;
200, occluder; 210, occlusion member; 211, distal end; 212, proximal end; 220, limiting member;
300, occluder; 310, occlusion member; 311, distal end; 312, proximal end; 320, limiting member; 330, hollow structure; and
400, occluder; 410, occlusion member; 411, distal end; 412, proximal end; 420, limiting member.

### DETAILED DESCRIPTION

The occluder and medical device proposed herein will be described in greater detail below combining with accompanying drawings and specific embodiments.
Features and advantages of the present application will be more apparent from the following detailed description, and the appended claims. It should be noted that the drawings are provided in a very simplified form not necessarily drawn to scale, for the only purpose to facilitate convenient and explicit description of embodiments of the present application. In particular, as the figures tend to have distinct emphases, they are often drawn to different scales.

All numeric values that appear in this document are presumed to be modified by the term "about", whether or not explicitly indicated. The term "about", in the context of a numeric value, generally refers to both the recited value and a range of neighboring values that one of skill in the art would consider equivalent to that value (i.e., having the same function or result). In many instances, the term "about" may include values that are rounded to the nearest significant figure. Other uses of the term "about" (i.e., in a context other than numeric values) may be assumed to have their ordinary and customary definition(s), as understood from and consistent with the context of the specification, unless otherwise specified.

As used herein, "proximal end" or "distal end" refers to the relative orientation, relative position, or direction of elements or actions that are relative to each other from the perspective of an operator operating the device. Yet without wishing to be limiting in any sense, the "proximal end" generally refers to the end of the medical device close to the operator during its normal operation, while the "distal end" generally refers to the end that enters into the body of the patient first.

The core concept of the present application is to provide an occluder and a medical device, the occluder including: an occlusion member having a distal end and a proximal end; and a limiting member connected to each of the distal and proximal ends and configured to limit an elongating distance between the distal and proximal ends. In particular, limiting member is configured to limit the elongating distance between the distal and proximal ends to a distance less than the distance between the distal and proximal ends in a fully-elongated configuration of the occlusion member. By the limiting member connected to the distal and proximal ends of the occlusion member to limit the elongating distance between the distal and proximal ends, the occluder will be in a partially- or fully-folded configuration when loaded in the sheath, avoiding the excessive in-sheath length of the occluder. Further, since the occluder in the sheath is not fully elongated and is in a partially or fully folded configuration, the dimension of the occluder after it is released from the sheath is better maintained.

Here, "full elongation" means that the occluder reaches its maximum length.At this moment, the occlusion member also reaches its maximum length as well as an elongation limit (where any additional elongation will lead to the failure of the contract/recoil), and the distance between the distal and proximal ends is the farthest. "Partial elongation" or "incomplete elongation" means that the occluder does not reach its maximum length. At this moment, the occlusion member does not reach its maximum length yet (i.e., there is still a margin/surplus for additional elongation that can be eliminated later by contraction/recoil), and the distance between the distal and proximal ends is not the farthest.

"Partial or full folding" means that the occluder does not reach its maximum length. At this moment, the occlusion member does not reach its maximum length yet (i.e., there is still a margin/surplus for additional elongation that can be eliminated later by contraction/recoil), and the distance between the distal and proximal ends is not the farthest. Meanwhile, the occlusion member is in a compressed configuration, i.e. the occlusion member approaches the axis thereof.

The present application will be described in greater detail below by way of a few particular examples.

### Embodiment 1

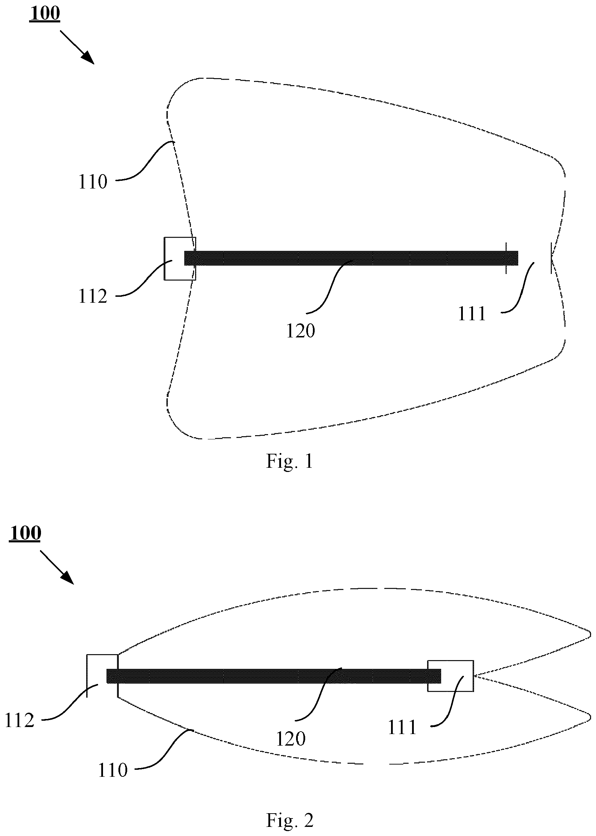

Referring to Fig 1, a schematic illustration of an occluder in a deployed configuration according to embodiment 1, which shares many features with the present invention but which is not in accordance with the claimed invention, and to Fig. 2, a schematic illustration of the occluder in a folded configuration according to embodiment 1. As shown in Figs. 1 and 2, the occluder 100 includes: an occlusion member 110 having a distal end 111 and a proximal end 112; and a limiting member 120 connected to each of the distal end 111 and proximal end 112 and configured to limit an elongating distance between the distal end 111 and proximal end 112. Here, the elongating distance refers to the distance between the distal end 111 and proximal end 112 after the occlusion member 110 is elongated. Due to the presence of the limiting member 120, the occlusion member 110 can only be partially or incompletely elongated and cannot reach a fully-elongated configuration.

The limiting member 120 may be connected to the distal end 111 and proximal end 112 by means of suturing, bonding, hot-melting, welding, threading or snapping.

The limiting member 120 is a flexible structure having opposing ends that are respectively connected to the distal end 111 and proximal end 112. In comparison with a rigid structure, the flexible structure is more compliant, so that the occluder 100, when released, is able to better fit the shape of the organ to be occluded to achieve a better occlusion to be organ. Specifically, the flexible structure may be selected from the metal wires (filaments/strings) or non-metallic wires (filaments/strings). For example, in an embodiment, the limiting member 120 is an implantable string. The limiting member 120 is connected to the distal and proximal ends 111, 112 by suturing.

The limiting member 120 may have a length that is determined as needed. Preferably, a maximum length of the limiting member 120 is greater than or equal to the distance between the distal and proximal ends 111, 112 in the deployed configuration of the occlusion member 110 and less than the distance between the proximal ends 111, 112 in the fully-elongated configuration of the occlusion member 110. Here, the maximum length of the limiting member 120 refers to a length of the limiting member 120 in a straightened state.

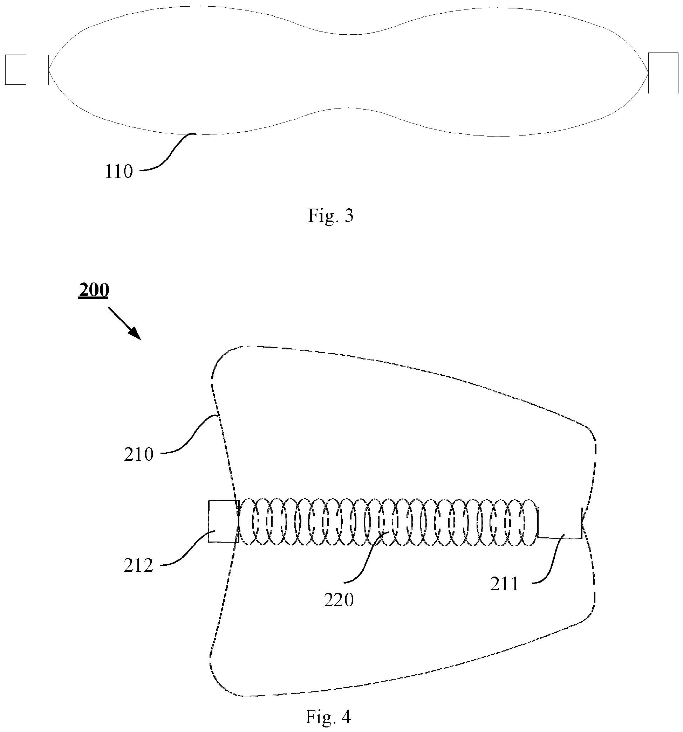

In the deployed configuration of the occlusion member 110, as shown in Fig. 1, the occlusion member 110 can be used to achieve the occlusion to an organ, such as a left atrial appendage (LAA). Specifically, when the occlusion member 110 is deployed, the occlusion member 110 expands towards the direction away from its axis and reaches its maximum radial dimension (where the occlusion member 110 cannot expand any more) outside the organ to be occluded. In the organ to be occluded, as limited by the shape of the organ to be occluded, the occlusion member 110 may have a radial dimension that is smaller than the maximum radial dimension and greater than a minimum radial dimension (where the opposing sides of the occlusion member 110 are brought into contact with each other) e. In general, the radial dimension of the occlusion member 110 is equal to, or slightly smaller than, the maximum radial dimension. In the fully-elongated configuration, as shown in Fig. 3, the occlusion member 110 has a maximum length.

In embodiments of present application, the occlusion member 110 includes a frame (not shown) covered with a membrane (not shown), which may be either permeable or non-permeable. The distal and proximal ends 111, 112 are located on the frame. Further, the frame may be provided thereon with anchors (not shown) for securing the occlusion member 110 to the organ to be occluded.

In this embodiment, the occluders of a same specification (the occluder with a limiting member and the occluder without a limiting member have occlusion members with the same structural, dimensional, material and other parameters) having a limiting member (here, the occluder only includes an occlusion member) or not having a limiting member (here, the limiting member is an implantable string) are tested by the simulated implantation process. The occluder with a limiting member presents a folded and compressed configuration (reference can be made to Fig. 2 accordingly) after loaded in the sheath. The occluder with a limiting member present no abnormality in shape after released, i.e. having a shape in consistent with the occluder without a limiting member.

In-sheath lengths and dimensional retentions of the two occluders were recorded and summarized below in Table 1.

**Table 1**

| Occluder | In-Sheath Length (mm) | Occluder Deformation after Release from Sheath |
|---|---|---|
| Occluder without a limiting member | 30 | 9% |
| Occluder with a limiting member | 22 | 4% |
| Reduction in In-sheath Length | 27% | / |

As can be seen from Table 1, the occluder having the limiting member (i.e., the occluder 100 having the limiting member of the implantable string) has a shorter in-sheath length and exhibits a less deformation after released. In other words, the occluder with the limiting member has a better dimensional retention performance.

Accordingly, there is also provided a medical device in this embodiment. The medical device includes a sheath (not shown) defining a delivery channel, and a the occluder 100 as discussed above, in which the occluder 100 can be delivered to a target site through the delivery channel of the sheath. When the occluder 100 is loaded in the sheath, the occlusion member in the occluder 100 is in a partially- or fully-folded configuration (see Fig. 2), with the limiting member 120 being straightened to limit the elongating distance between the distal and proximal ends 111, 112 of the occlusion member 110. When the occluder 100 is released from the sheath, the occlusion member 110 is in a deployed configuration (see Fig. 1), and the limiting member 120 is in a loose state.

By the limiting member 120 connected to the distal and proximal ends 111, 112 of the occlusion member 110 to limit the elongating distance between the distal and proximal ends 111, 112, the occluder will be in a partially- or fully-folded configuration when loaded in the sheath, avoiding the excessive in-sheath length of the occluder 100. Further, since the occluder 100 in the sheath is not fully elongated and is in a partially or fully folded configuration, the dimension of the occluder 100 after it is released from the sheath is better maintained.

### Embodiment 2

In embodiment 2, which does not fall within the scope of the present invention but which shares many features with the present invention, there are provided an occluder and a medical device, the limiting member in the occluder being a flexible structure. The embodiment 2 differs from the embodiment 1 in that the limiting member is a nickel-titanium alloy wire and connected to distal and proximal ends of an occlusion member via welding. Therefore, specific structures of occluder and medical device in this embodiment are omitted, which can be referred to the description of embodiment 1.

In this embodiment, the occluders of a same specification (the occluder with a limiting member and the occluder without a limiting member have occlusion members with the same structural, dimensional, material and other parameters) having a limiting member (here, the occluder includes only an occlusion member) or not having a limiting member (here, the limiting member is a nickel-titanium alloy wire) are tested by the simulated implantation process. The occluder with a limiting member presents a folded and compressed configuration (reference can be made to Fig.2 accordingly) after loaded in the sheath. The occluder with a limiting member present no abnormality in shape after released, i.e. having a shape in consistent with the occluder without a limiting member.

In-sheath lengths and dimensional retentions of the two occluders were recorded and summarized below in Table 2.

**Table 2**

| Occluder | In-Sheath Length (mm) | Occluder Deformation after Release from Sheath |
|---|---|---|
| Occluder without a limiting member | 30 | 9% |
| Occluder with a limiting member | 21 | 7% |
| Reduction in In-sheath Length | 30% | / |

As can be seen from Table 2, the occluder having the limiting member (i.e., the occluder having the limiting member of the nickel-titanium alloy wire) has a shorter in-sheath length and exhibits a less deformation after released. In other words, the occluder with the limiting member has a better dimensional retention performance.

### Embodiment 3

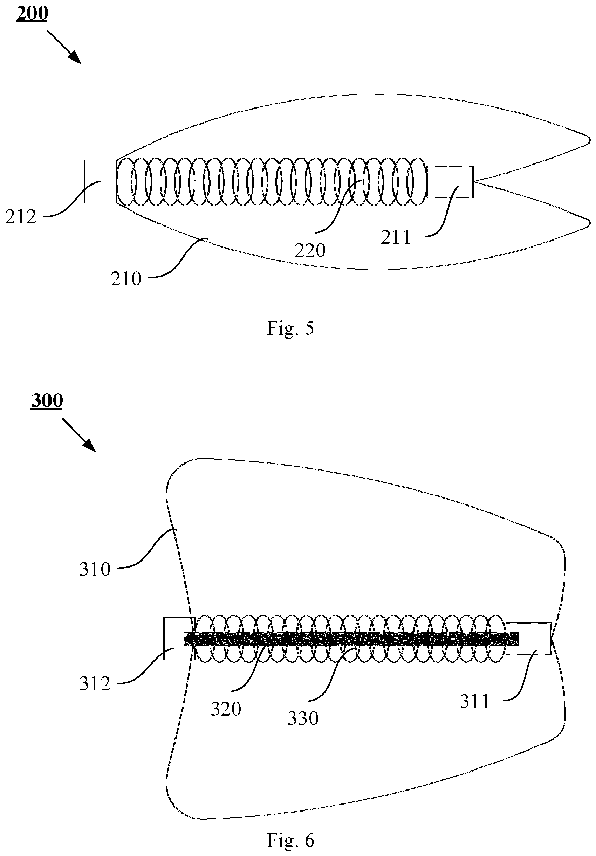

Reference is now made to Fig. 4, a schematic illustration of an occluder in a deployed configuration according to embodiment 3 which shares many features with the present application but which does not fall within the scope of the claimed invention, and to Fig. 5, a schematic illustration of the occluder in a folded configuration according to embodiment 3. As shown in Figs. 4 and 5, the occluder 200 includes: an occlusion member 210 having a distal end 211 and a proximal end 212; and a limiting member 220 connected to the distal and proximal
ends 111, 112 and configured to limit an elongating distance between the distal and proximal ends 211, 212.

The limiting member 220 may be connected to the distal and proximal ends 211, 212 by means of suturing, bonding, hot-melting, welding, threading or snapping.

In this embodiment, the limiting member 220 is an elastic structure having opposing ends that are respectively connected to the distal and proximal ends 211, 212. Specifically, the elastic structure is an elastic string or a spring. For example, in this embodiment, the limiting member 220 is an implantable spring and is connected to the distal and proximal ends 211, 212 through welding.

The limiting member 220 may have a length that is determined as needed. Preferably, a maximum permissible length of the limiting member 220 is greater than or equal to the distance between the distal and proximal ends 211, 212 in the deployed configuration of the occlusion member 210 and less than the distance between the distal and proximal ends 211, 212 in the fully-elongated configuration of the occlusion member 210. Here, the maximum length of the limiting member 220 refers to a length of the limiting member 220in a straightened state, i.e., a length of the limiting member 220 in a most elongated state (where it is elastically deformed to a maximum extent).

In this embodiment, a minimum length of the limiting member 220 is less than or equal to the distance between the distal and proximal ends 211, 212 of the limiting member 220 in the deployed configuration of the occlusion member 210. In other words, the length of the limiting member 220 that is not elongated (i.e., not elastically deformed at all) is less than or equal to the distance between the distal and proximal ends 211, 212 of the limiting member 220 in the deployed configuration of the occlusion member 210.

Reference can be made to the description of the occlusion member of the embodiment 1 for more details in the occlusion member of the embodiment 3, and a further description thereof is omitted. Accordingly, there is also provided a medical device in embodiment 3. Likewise, reference can be made to the description of the medical device of embodiment 1 for more details in the medical device of embodiment 3, and a further description thereof is omitted.

In this embodiment, the occluders of a same specification (the occluder with a limiting member and the occluder without a limiting member have occlusion members with the same structural, dimensional, material and other parameters) having a limiting member (here, the occluder includes only an occlusion member) or not having a limiting member (here, the limiting member is a spring) are tested by the simulated implantation process. The occluder with a limiting member presents a folded and compressed configuration (reference can be made to Fig.5 accordingly) after loaded in the sheath. The occluder with a limiting member presents no abnormality in shape after released, i.e. having a shape in consistent with the occluder without a limiting member.

In-sheath lengths and dimensional retentions of the two occluders were recorded and summarized below in Table 3.

**Table 3**

| Occluder | In-Sheath Length (mm) | Occluder Deformation after Release from Sheath |
|---|---|---|
| Occluder without a limiting member | 30 | 9% |
| Occluder with a limiting member | 21 | 4% |
| Reduction in In-sheath Length | 30% | / |

As can be seen from Table 3, the occluder having the limiting member (i.e., the occluder 200 having the limiting member of the spring) has a shorter in-sheath length and exhibits a less deformation after released. In other words, the occluder with the limiting member has a better dimensional retention performance.

### Embodiment 4

In embodiment 4, which shares many features with the present invention but which does not fall within the scope of the claimed invention, there are provided an occluder and a medical device, the limiting member in the occluder being an elastic structure. The embodiment 4 differs from the embodiment 3 in that the limiting member is an implantable elastic string and connected to distal and proximal ends of an occlusion member by welding. Therefore, reference can be made to the description of embodiment 3 for more details in the occluder and medical device of embodiment 4.

In this embodiment, the occluders of a same specification (the occluder with a limiting member and the occluder without a limiting member have occlusion members with the same structural, dimensional, material and other parameters) having a limiting member (here, the occluder includes only an occlusion member) or not having a limiting member (here, the limiting member is an implantable elastic string) are tested by the simulated implantation process. The occluder with a limiting member presents a folded and compressed configuration (reference can be made to Fig. 5 accordingly) after loaded in the sheath. The occluder with a limiting member present no abnormality in shape after released, i.e. having a shape in consistent with the occluder without a limiting member.

In-sheath lengths and dimensional retentions of the two occluders were recorded and summarized below in Table 4.

**Table 4**

| Occluder | In-Sheath Length (mm) | Occluder Deformation after Release from Sheath |
|---|---|---|
| Occluder without a limiting member | 30 | 9% |
| Occluder with a limiting | 21 | 4% |
| member | | |
| Reduction in In-sheath Length | 30% | / |

As can be seen from Table 4, the occluder having the limiting member (i.e., the occluder having the limiting member of the implantable elastic string) has a shorter in-sheath length and exhibits a less deformation after released. In other words, the occluder with the limiting member has a better dimensional retention performance.

### Embodiment 5

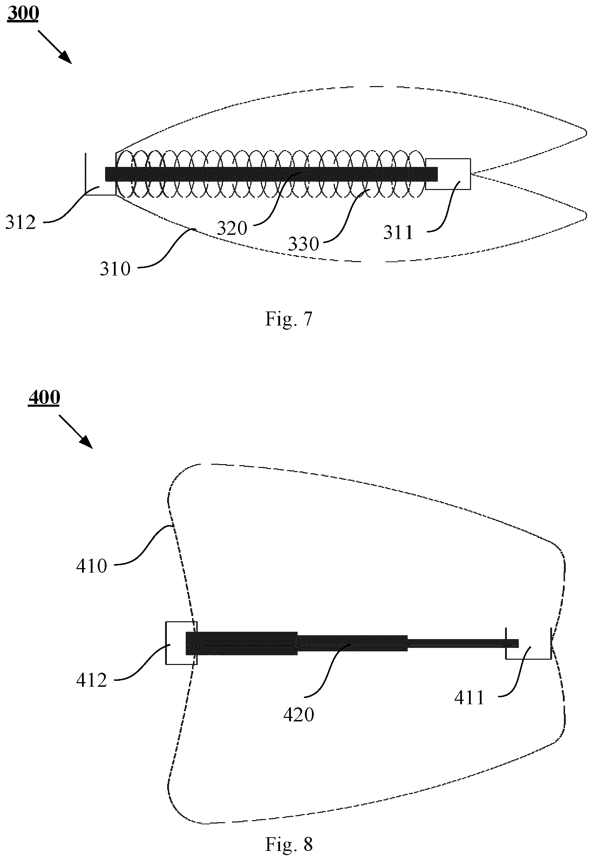

Reference is now made to Fig. 6, a schematic illustration of an occluder in a deployed configuration according to embodiment 5, which shares many features with the present invention but which does not fall within the scope of the claimed invention, and to Fig. 7, a schematic illustration of the occluder in a partially folded configuration according to embodiment 5. As shown in Figs. 6 and 7, the occluder 300 includes: an occlusion member 310 having a distal end 311 and a proximal end 312; and a limiting member 320 connected to the distal and proximal ends 311, 312 and configured to limit an elongating distance between the distal and proximal ends 311, 312.

The limiting member 320 may be connected to the distal and proximal ends 311, 312 by means of suturing, bonding, hot-melting, welding, threading or snapping.

The limiting member 320 is a flexible structure having opposing ends that are respectively connected to the distal and proximal ends 311, 312. Specifically, the elastic structure may be selected from metal wires (filaments /strings) or non-metallic wires (filaments /strings). For example, in this embodiment, the limiting member 320 is an implantable string. The limiting member 320 is connected to the distal and proximal ends 311, 312 by suturing

The limiting member 320 may have a length that is determined as needed. Preferably, a maximum length of the limiting member 320 is greater than or equal to the distance between the distal and proximal ends 311, 312 in the deployed configuration of the occlusion member 310 and less than the distance between the distal and proximal ends 311, 312 in the fully-elongated configuration of the occlusion member 310. Here, the maximum length of the limiting member 320 refers to a length of the limiting member 320 in a straightened state. When the length of the limiting member 320 is equal to or slight greater than the distance between the distal and proximal ends 311, 312 in the deployed configuration of the occlusion member 310, the occlusion member 310 presents a fully-folded configuration in the sheath. In this embodiment, the occluder 300 further includes a hollow structure 330, the limiting member 320 being located in the chamber of the hollow structure 330, and the hollow structure 330 being located in the occlusion member 310. the limiting member 320 being located in the chamber of the hollow structure 330 means that the hollow structure 330 may wrap around the limiting member 320. In this way, when the occluder 300 is released from the sheath, and the limiting member 320 is in a loose state, the limiting member 320 can be confined within the hollow structure 330 to avoid the leakage of the limiting member 320 from the occlusion member 310. Thus, a higher reliability of the occluder 300 is obtained.

Preferably, the hollow structure 330 may be a spring or a telescopic tube. A maximum length (i.e., a maximum elongation length) of the hollow structure 330 may be greater than the maximum length of the limiting member 320. In other words, when the occlusion member 310 is folded (i.e., being partially and incompletely elongated) in the sheath, the limiting member 320 is tightened to be straight, and the hollow structure 330 may be in a non-straightened state. Here, the elongating distance between the distal and proximal ends 311, 312 of the occlusion member 310 is restricted only by the limiting member 320. In an alternative embodiment, the maximum length (or maximum elongation length) of the hollow structure 330 may be equal to the maximum length of the limiting member 320. That is to say, the elongating distance between the distal and proximal ends 311, 312 of the occlusion member 310 is restricted by both the limiting member 320 and the hollow structure 330.

Reference can be made to the description of the occlusion member of embodiment 1 for more details in the occlusion member of embodiment 5, and a further description thereof is omitted. Accordingly, there is also provided a medical device in embodiment 5. Likewise, reference can be made to the description of the medical device of embodiment 1 for more details in the medical device of embodiment 5, and a further description thereof is omitted.

In this embodiment, the occluders of a same specification (the occluder with a limiting member and the occluder without a limiting member have occlusion members with the same structural, dimensional, material and other parameters) having a limiting member (here, the occluder only includes an occlusion member) or not having a limiting member (here, the limiting member is an implantable string) are tested by the simulated implantation process. The occluder with a limiting member presents a folded and compressed configuration (reference can be made to Fig. 7 accordingly) after loaded in the sheath. The occluder with a limiting member present no abnormality in shape after released, i.e. having a shape in consistent with the occluder without a limiting member.

In-sheath lengths and dimensional retentions of the two occluders were recorded and summarized below in Table 5.

**Table 5**

| Occluder | In-Sheath Length (mm) | Occluder Deformation after Release from Sheath |
|---|---|---|
| Occluder without a limiting | 30 | 9% |
| member | | |
| Occluder with a limiting member | 21 | 3% |
| Reduction in In-sheath Length | 30% | / |

As can be seen from Table 5, the occluder having the limiting member (i.e., the occluder having the limiting member of the implantable string) has a shorter in-sheath length and exhibits a less deformation after released. In other words, the occluder with the limiting member has a better dimensional retention performance.

### Embodiment 6

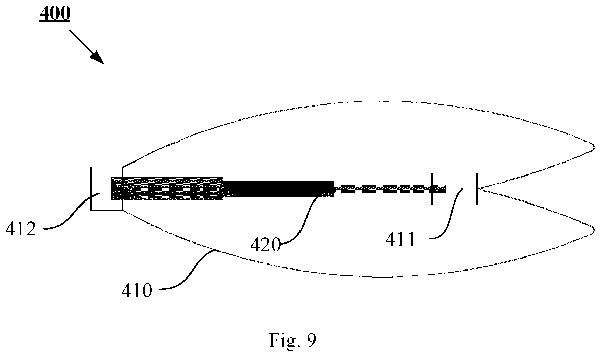

Reference is now made to Fig. 8, a schematic illustration of an occluder in a deployed configuration according to embodiment 6 of the present application, and to Fig. 9, a schematic illustration of the occluder in a folded configuration according to embodiment 6 of the present application. As shown in Figs. 8 and 9, the occluder 400 includes: an occlusion member 410 having a distal end 411 and a proximal end 412; and a limiting member 420 connected to the distal and proximal ends 411, 412 and configured to limit an elongating distance between the distal and proximal ends 411, 412. Here, the elongating distance refers to the distance between the distal and proximal ends 411, 412 in an elongated state of the occlusion member 410. Due to the presence of the limiting member 420, the occlusion member 410 can only be partially or incompletely elongated and cannot reach a fully-elongated configuration.

The limiting member 420 may be connected to the distal and proximal ends 411, 412 by means of suturing, bonding, hot-melting, welding, threading or snapping.

In this embodiment, the limiting member 420 is a telescopic structure having opposing ends that are respectively connected to the distal and proximal ends 411, 412. Specifically, the telescopic structure may be a nesting tube formed by multiple metal tube sections that are nested one into another. The length of the limiting member 420 can be changed by stretching and retracting the metal tube sections.

The limiting member 420 may have a length that is determined as needed. Preferably, a maximum length of the limiting member 120 is greater than or equal to the distance between the distal and proximal ends 411, 412 in the deployed configuration of the occlusion member 410 and less than the distance between the proximal ends 411, 412 in the fully-elongated configuration of the occlusion member 410. Here, the maximum length of the limiting member 420 refers to a length of the limiting member 420 in a straightened state, and also refers to the length of the limiting member 420 in the state that all the metal tube sections are fully stretched.

In this embodiment, a minimum length of the limiting member 420 is less than or equal to the distal and proximal ends 411, 412 in the deployed configuration of the occlusion member 410. Here, the minimum length of the limiting member 420 refers to a length of limiting member 420 in the state that the metal tube sections are fully contracted, i.e., the state that one metal tube section nests remaining metal tube sections therein.

Further, the occluder 400 also includes at least one deflecting member (not shown), through which at least one end of the limiting member 420 is connected to the distal end 411 or proximal end 412. For example, one end of the limiting member 420 is connected to the distal end 411 via the deflecting member, with the other end of the limiting member 420 being directly connected to the proximal end 412. For another example, one end of the limiting member 420 is directly connected to the distal end 411, with the other end of the limiting member 420 being connected to the distal end 411 via the deflecting member. For still another example, one end of the limiting member 420 is connected to the distal end 411 via such a deflecting member, with the other end of the limiting member 420 being connected to the proximal end 412 via another deflecting member. The deflecting member(s) is able to control an orientation of the distal end 411 of the occlusion member 410 so that the occluder 400 can be more easily released from the sheath.

Reference can be made to the description of the occlusion member of embodiment 1 for more details in the occlusion member of embodiment 6, and a further description thereof is omitted. Accordingly, there is also provided a medical device in embodiment 6. Likewise, reference can be made to the description of the medical device of embodiment 1 for more details in the medical device of embodiment 6, and a further description thereof is omitted.

Other embodiments can be obtained from combinations of the foregoing embodiments. For example, the occluder of the embodiment 4 may also include deflecting member(s), through which at least one end of the elastic structure that serves as the limiting member is connected to the distal or proximal end of the occlusion member.

## Claims

1. An occluder (400), comprising:
an occlusion member (410) having a distal end (411) and a proximal end (412); and
a limiting member (420) connected to each of the distal and proximal ends and configured to limit an elongating distance between the distal and proximal ends, **characterized in that**, the limiting member is a telescopic structure and has its two ends connected to the distal and proximal ends respectively, and wherein the telescopic structure is a nested tube formed by multiple metal tube sections that are nested one into another.

2. The occluder (400) of claim 1, wherein the occluder further comprises a hollow structure, the limiting member (420) being located in a chamber of the hollow structure, the hollow structure being located in the occlusion member (410).

3. The occluder (400) of claim 2, wherein the hollow structure is a spring or a telescopic tube.

4. The occluder (400) of claim 1, wherein the occluder further comprises at least one deflecting member, through which at least one end of the limiting member (420) is connected to the distal end (411) or proximal end (412).

5. The occluder (400) of any one of claims 1 to 4, wherein the limiting member (420) is connected to the distal (411) and proximal (412) ends by means of suturing, bonding, hot-melting, welding, threading or snapping.

6. The occluder (400) of any one of claims 1 to 4, wherein the occlusion member (410) comprises a frame covered with a membrane, and wherein the distal (411) and proximal (412) ends are located on the frame.

7. A medical device, comprising:
a sheath defining a delivery channel; and
the occluder (400) of any one of claims 1 to 6, wherein the occluder is able to reach a target site through the delivery channel, wherein when the occluder is loaded in the sheath, the occlusion member (410) of the occluder is in a partially or fully folded configuration, and the limiting member (420) of the occluder is straightened to limit the elongating distance between the distal (411) and proximal (412) ends of the occlusion member, and wherein when the occluder is released from the sheath, the occlusion member is in an deployed configuration, and the limiting member is in an unconstrained state.

## Patentansprüche

1. Okkluder (400), Folgendes umfassend:
ein Okklusionselement (410), das ein distales Ende (411) und ein proximales Ende (412) aufweist; und
ein Begrenzungselement (420), das sowohl mit dem distalen als auch mit dem proximalen Ende verbunden und so konfiguriert ist, dass es eine Verlängerungsentfernung zwischen dem distalen und dem proximalen Ende begrenzt, **dadurch gekennzeichnet, dass** das Begrenzungselement eine teleskopische Struktur ist und seine beiden Enden jeweils mit dem distalen und dem proximalen Ende verbunden sind, und wobei die teleskopische Struktur ein verschachteltes Rohr ist, das durch mehrere Metallrohrabschnitte gebildet wird, die ineinander geschachtelt sind.

2. Okkluder (400) nach Anspruch 1, wobei der Okkluder ferner eine Hohlstruktur umfasst, wobei das Begrenzungselement (420) in einer Kammer der Hohlstruktur angeordnet ist, wobei die Hohlstruktur in dem Okklusionselement (410) angeordnet ist.

3. Okkluder (400) nach Anspruch 2, wobei die Hohlstruktur eine Feder oder ein teleskopisches Rohr ist.

4. Okkluder (400) nach Anspruch 1, wobei der Okkluder ferner mindestens ein Ablenkelement umfasst, durch das mindestens ein Ende des Begrenzungselements (420) mit dem distalen Ende (411) oder dem proximalen Ende (412) verbunden ist.

5. Okkluder (400) nach einem der Ansprüche 1 bis 4, wobei das Begrenzungselement (420) mit dem distalen (411) und dem proximalen (412) Ende durch Nähen, Kleben, Heißschmelzen, Schweißen, Verschrauben oder Einschnappen verbunden ist.

6. Okkluder (400) nach einem der Ansprüche 1 bis 4, wobei das Okklusionselement (410) einen mit einer Membran bedeckten Rahmen umfasst, und wobei das distale (411) und das proximale (412) Ende an dem Rahmen angeordnet sind.

7. Medizinische Vorrichtung, Folgendes umfassend:
eine Hülle, die einen Einführungskanal definiert; und
den Okkluder (400) nach einem der Ansprüche 1 bis 6, wobei der Okkluder in der Lage ist, eine Zielstelle durch den Zuführkanal zu erreichen, wobei, wenn der Okkluder in die Hülle geladen ist, das Okklusionselement (410) des Okkluders in einer teilweise oder vollständig gefalteten Konfiguration ist, und das Begrenzungselement (420) des Okkluders gestreckt ist, um die Verlängerungsentfernung zwischen dem distalen (411) und dem proximalen (412) Ende des Okkluders zu begrenzen, und wobei, wenn der Okkluder aus der Hülle freigegeben wird, das Okklusionselement in einer entfalteten Konfiguration ist und das Begrenzungselement in einem unbelasteten Zustand ist.

## Revendications

1. Dispositif d'occlusion (400) comprenant :
un élément d'occlusion (410) ayant une extrémité distale (411) et une extrémité proximale (412), et
un élément de limitation (420) relié à chacune des extrémités distale et proximale et configuré pour limiter une distance d'allongement entre les extrémités distale et proximale ; **caractérisé en ce que** l'élément de limitation est une structure télescopique dont les deux extrémités sont respectivement reliées aux extrémités distale et proximale, et dans lequel la structure télescopique est un tube emboîté formé par plusieurs sections de tube métallique qui sont emboîtées les unes dans les autres.

2. Dispositif d'occlusion (400) selon la revendication 1, dans lequel le dispositif d'occlusion comprend en outre une structure creuse, l'élément de limitation (420) étant situé dans une chambre de la structure creuse, la structure creuse étant située dans l'élément d'occlusion (410).

3. Dispositif d'occlusion (400) selon la revendication 2, dans lequel la structure creuse est un ressort ou un tube télescopique.

4. Dispositif d'occlusion (400) selon la revendication 1, dans lequel le dispositif d'occlusion comprend en outre au moins un élément de déflexion, à travers lequel au moins une extrémité de l'élément de limitation (420) est reliée à l'extrémité distale (411) ou à l'extrémité proximale (412).

5. Dispositif d'occlusion (400) selon l'une quelconque des revendications 1 à 4, dans lequel l'élément de limitation (420) est relié aux extrémités distale (411) et proximale (412) par suture, collage, fusion à chaud, soudage, enfilage ou encliquetage.

6. Dispositif d'occlusion (400) selon l'une quelconque des revendications 1 à 4, dans lequel l'élément d'occlusion (410) comprend un cadre recouvert d'une membrane, et dans lequel les extrémités distale (411) et proximale (412) sont situées sur le cadre.

7. Dispositif médical, comprenant :
une gaine définissant un canal de distribution, et le dispositif d'occlusion (400) selon l'une quelconque des revendications 1 à 6, dans lequel le dispositif d'occlusion est capable d'atteindre un site cible à travers le canal de distribution, dans lequel, lorsque le dispositif d'occlusion est chargé dans la gaine, l'élément d'occlusion (410) du dispositif d'occlusion se trouve dans une configuration partiellement ou entièrement pliée et l'élément de limitation (420) du dispositif d'occlusion est détordu pour limiter la distance d'allongement entre les extrémités distale (411) et proximale (412) de l'élément d'occlusion, et dans lequel, lorsque le dispositif d'occlusion est libéré de la gaine, l'élément d'occlusion se trouve dans une configuration déployée et l'élément de limitation dans un état non contraint.
